# EUROPEAN PATENT SPECIFICATION

(11) **EP 1 602 662 B1**
(45) Date of publication and mention of the grant of the patent: **23.04.2014**
(21) Application number: 04703805.4
(22) Date of filing: 21.01.2004
(51) Int. Cl.: C07J 41/00, C07J 21/00, C07J 7/00

(54) **METHOD OF OBTAINING 17ALPHA-ACETOXY-11BETA-(4-N,N-DIMETHYLAMINOPHENYL)-19-NORPREGNA-4,9-DIENE-3,20-DIONE**
VERFAHREN ZUR GEWINNUNG VON 17ALPHA-ACETOXY-11BETA-(4-N,N-DIMETHYLAMINOPHENYL)-19-NORPREGNA-4,9-DIEN-3,20-DION
PROCEDE D'OBTENTION DE 17ALPHA-ACETOXY-11BETA-(4-N,N-DIMETHYLAMINOPHENIL)-19-NORPREGNA-4,9-DIENE-3,20-DIONE

(30) Priority: 22.01.2003 ES 200300162
(43) Date of publication of application: 07.12.2005
(60) Divisional application: 10165052.1
(73) Proprietor: Crystal Pharma S.A.U, 47151 Boecillo (ES)
(72) Inventor: SILVA GUISASOLA, Luis Octavio, E-47151 Boecillo Valladolid (ES); GUTIERREZ FUENTES, Luis Gerardo, E-47151 Boecillo Valladolid (ES); ROSON NINO, Carlos, E-47151 Boecillo Valladolid (ES)
(74) Representative: ABG Patentes, S.L.
(86) International application number: PCT/ES2004/000026
(87) International publication number: WO 2004/065405

(56) References cited:
- WO-A-01/47945
- WO-A-01/74840
- WO-A-96/30390
- WO-A-99/45022
- US-A- 4 954 490
- RAO P.N. ET AL.: 'A practical large-scale synthesis of 17alpha-acetoxy-11béta-(4-N,N-dimethylamino phenyl)-19-norpregna-4,9-diene-3,20-dione (CDB-2914)' STEROIDS vol. 65, 2000, pages 395 - 400, XP004222665
- BRITTAIN H G ED - BRITTAIN H G: "POLYMORPHISM IN PHARMACEUTICAL SOLIDS", 1 January 1999 (1999-01-01), POLYMORPHISM IN PHARMACEUTICAL SOLIDS; [DRUGS AND THE PHARMACEUTICAL SCIENCES ; 95], MARCEL DEKKER INC, NEW YORK * BASEL, PAGE(S) 199-200 ISBN: 978-0-8247-0237-3
- Rong Liu: "Water insoluble drug formulation", 2000, Interpharm/CRC, Boca Raton, Florida, USA ISBN: 9781574911053 pages 545-546,
- "From a crystalline solvate: Desolvation/Dehydration" In: HILFIKER R: "POLYMORPHISM IN THE PHARMACEUTICAL INDUSTRY", 1 January 2006 (2006-01-01), WILEY VCH VERLAG ISBN: 9783527311460 pages 268-268,
- SWABRICK J ET AL: ENCYCLOPEDIA OF PHARMACEUTICAL TECHNOLOGY, GENETIC ENGINEERING TO HYDROGELS, vol. 18, 1 January 1999 (1999-01-01), pages 398-398,
- Yihong Qiu et al: "Developing Solid Oral Dosage Forms: Pharmaceutical Theory and Practice", 2009, Academic Press, London, UK ISBN: 9780444532428 pages 42-42,
- Juan J. Novoa et al: "Engineering of crystalline materials' properties: state of the art in modeling, design and applications", 2007, Springer Verlag, Berlin, DE ISBN: 9781402068232 pages 432-432,
- ADDISON AULT: "TECHNIQUES AND EXPERIMENTS FOR ORGANIC CHEMISTRY, 6th Edition", 1 January 1998 (1998-01-01), University Science Books, Sausalito, California, USA ISBN: 9780935702767 pages 48-48,
- John Kenkel: "Analytical Chemistry for Technicians", 2002, Interpharm/CRC, Boca Raton, Florida, USA ISBN: 9781566705196 pages 322-322,
- VOTANO JOSEPH R ET AL: "Three new consensus QSAR models for the prediction of Ames genotoxicity.", MUTAGENESIS SEP 2004, vol. 19, no. 5, September 2004 (2004-09), pages 365-377, ISSN: 0267-8357
- "Guidance for Industry, Genotoxic and Carcinogenic Impurities in Drug Substances and Products: Recommended Approaches [DRAFT GUIDANCE, 1:\7834dft.doc]", 3 December 2008 (2008-12-03), US Department of Health and Human Services, Food and Drug Administration, Center for Drug Evaluation and Research (CDER), Rockville, MD, USA pages 1-13,
- "ICH Harmonised Tripartite Guideline: Impurities in New Drug Substances Q3A (R2)", 25 October 2006 (2006-10-25), ICH Expert Working Group pages 1-11,
- DELANEY ET AL: "An impact analysis of the application of the threshold of toxicological concern concept to pharmaceuticals", REGULATORY TOXICOLOGY AND PHARMACOLOGY, ACADEMIC PRESS,NEW YORK, NY, US, vol. 49, no. 2, 30 October 2007 (2007-10-30), pages 107-124, XP022322196, ISSN: 0273-2300, DOI: 10.1016/J.YRTPH.2007.06.008
- Wilfred L F Armarego; Christina Li Lin Chai: "Purification of laboratory chemicals, 6th Ed", 1 January 2009 (2009-01-01), ELSEVIER, AMSTERDAM, NL ISBN: 978-1-85617-567-8 pages 14-17,
- Committee for Medicinal Products for Human Use (CHMP): "Guideline on the Limits of Genotoxic Impurities", 28 June 2006 (2006-06-28), European Medicines Agency, London, UK pages 1-8,

## Description

### FIELD OF THE INVENTION

The invention relates to a process for obtaining 17α-acetoxy-11β-(4-N,N-dimethylaminophenyl)-19-norpregna-4,9-diene-3,20-dione from its isopropanol hemisolvate.

### BACKGROUND OF THE INVENTION

Compound 17α-acetoxy-11β-(4-N,N-dimethylaminophenyl)-19-norpregna-4,9-diene-3,20-dione, hereinafter VA-2914, with formula 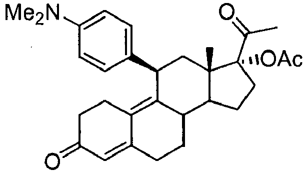 is a known steroid with anti-progestational and anti-glucocorticoid activity which is useful in therapeutic and contraceptive gynaecological indications (uterine fibromas, endometriosis) and for treating Cushing syndrome and glaucoma. Said compound, as well as a process for obtaining it, is described in US patent 4,954,490.

An alternative synthesis of VA-2914 is disclosed in US patent 5,929,262. The final product obtained by means of the process described in Example 7 of said US patent 5,929,262 is described as a product in the form of yellow crystals with a melting point between 183°C and 185°C. The existence of said yellow colour indicates the presence of impurities, mainly phenol compounds.

It has now been found that VA-2914 isopropanol hemisolvate, which can be obtained with a high purity, is a useful intermediate in preparing VA-2914. Recrystallisation of an appropriate solvent for said isopropanol hemisolvate, such as ethanol/water or ethyl ether, gives a white coloured VA-2914, indicating the high degree of purity obtained.

Therefore, an aspect of the present invention is related to a process for purifying VA-2914 comprising obtaining VA-2914 isopropanol hemisolvate and converting it into VA-2914.

The VA-2914 isopropanol hemisolvate has been identified and characterised by its infrared (IR) spectrum, its exotherm by differential scanning calorimetry (DSC) and its X-ray diffractogram (XRD). The isopropanol content (about 5.9%) in said VA-2914 isopropanol hemisolvate was determined by gas chromatography and analysed by means of the internal standard technique.

The VA-2914 isopropanol hemisolvate can be obtained from VA-2914 and isopropanol.

The VA-2914 isopropanol hemisolvate can be used in obtaining VA-2914 or in purifying raw VA-2914. The raw VA-2914 compound may be obtained by methods known in the state of the art. However, in a particular embodiment, raw VA-2914 compound is obtained from compound 3,3-(1,2-ethanedioxy)-5α-hydroxy-11β-(4-N,N-dimethylaminophenyl)-17α-acetoxy-19-norpregna-9-ene-20-one [carbinol acetate], which constitutes an additional aspect of this invention.

### BRIEF DESCRIPTION OF THE DRAWINGS

Figure 1 is a graph showing the infrared (IR) absorbance spectrum for the VA-2914 isopropanol hemisolvate crystalline form performed with a potassium bromide pellet in a Perkin Elmer 1600 Fourier Transform (FT_IR) IR spectrophotometer. Transmittance is shown on the Y axis and wave number on the X axis (cm⁻¹).
Figure 2 is a graph showing exotherm by differential scanning calorimetry (DSC) for the VA-2914 isopropanol hemisolvate crystalline form. The energy absorbed or transmitted per time unit (mW) is shown on the Y axis, and the temperature (C°) and time (minutes) are shown on the X axis.
Figure 3 is a graph showing the powder X-ray diffraction (XRD) spectrum of the VA-2914 isopropanol hemisolvate crystalline form obtained with a radiation source with a wavelength α₁ of 1.54060 Angstroms (Å), a wavelength α₂ of 1.54439 Å, an intensity ratio α₁/α₂ of 0.5, 40 kV voltage and 30 mA of current intensity, in Debye-Scherrer INEL CPS-120 equipment. The Y axis shows the pulses and in el X axis the angle 2θ.
Figure 4 is a graph showing the infrared (IR) absorbance spectrum of the VA-2914 crystalline form obtained by recrystallisation in ethyl ether or in ethanol/water [US 5,929,262], performed with a potassium bromide pellet, in a Perkin Elmer 1600 Fourier Transform IR (FT_IR) IR spectrophotometer. Transmittance is shown on the Y axis and wave number on the X axis (cm⁻¹).
Figure 5 is a graph showing exotherm by differential scanning calorimetry (DSC) for the VA-2914 crystalline form obtained by recrystallisation in ethyl ether or in ethanol/water [US 5,929,262]. The energy absorbed or transmitted per time unit (mW) is shown on the Y axis, and the temperature (C°) and time (minutes) are shown on the X axis.
Figure 6 is a graph showing the powder X-ray diffraction (XRD) spectrum of the VA-2914 crystalline form obtained by recrystallisation in ethyl ether or in ethanol/water [US 5,929,262], obtained with a radiation source with a wavelength α₁ of 1.54060 Angstroms (Å), a wavelength α₂ of 1.54439 Å, an intensity ratio α₁/α₂ of 0.5, 40 kV voltage and 30 mA of current intensity, in Debye-Scherrer INEL CPS-120 equipment. The Y axis shows the pulses and in el X axis the angle 2θ.
Figure 7 is a graph showing exotherm by differential scanning calorimetry (DSC) for (i) the VA-2914 crystalline form obtained by recrystallisation in ethyl ether or in ethanol/water [US 5,929,262], and (ii) the VA-2914 isopropanol hemisolvate crystalline form. The energy absorbed or transmitted per time unit (mw) is shown on the Y axis, and the temperature (C°) and time (minutes) are shown on the X axis.

### DETAILED DESCRIPTION OF THE INVENTION

### Obtaining VA-2914

In a first aspect the present invention provides a process for purifying VA-2914 comprising its recristallisation in isopropanol under conditions allowing obtaining, in an unequivocal and reproducible manner, a VA-2914 crystalline form, specifically its isopropanol hemisolvate, useful as an intermediate in obtaining highly pure VA-2914.

More specifically, the process for purifying VA-2914 provided by this invention comprises:
a) forming VA-2914 isopropanol hemisolvate crystals by means of crystallising VA-2914 in isopropanol;
b) separating the VA-2914 isopropanol hemisolvate crystals by filtration; and
c) converting the VA-2914 isopropanol hemisolvate into VA-2914.

The VA-2914 compound can be obtained by means of any of the processes known in the state of the art [see, for example, US patent 4,954,490 and US patent 5,929,262]. However, in a particular embodiment, said VA-2914 can be obtained from compound 3,3-(1,2-ethanedioxy)-5α-hydroxy-11β-(4-N,N-dimethylaminophenyl)-17α-acetoxy-19-norpregna-9-ene-20-one, called carbinol acetate in this description, by means of a process comprising deprotecting the ketone and dehydrating said compound in order to obtain VA-2914, for example, by means of acid hydrolysis. In principle, any organic or inorganic acid capable of hydrolysing the cetal group and removing the hydroxyl group present in position 5 can be used, for example, sulphuric acid, trifluoroacetic acid, monopotassium sulfate, etc.

VA-2914 isopropanol hemisolvate crystal formation by crystallising VA-2914 in isopropanol comprises previous dissolution of VA-2914 in isopropanol and subsequent formation of said VA-2914 isopropanol hemisolvate crystals. Dissolution of VA-2914 in isopropanol is preferably carried out under heat, which facilitates its dissolution, and then the resulting solution is allowed to cool, optionally under stirring, so that the VA-2914 isopropanol hemisolvate crystals form. In a particular embodiment, the VA-2914 and isopropanol mixture is heated at a temperature comprised between 75°C and the reflux temperature of the solvent, up to complete dissolution of VA-2914, and the solution of VA-2914 in isopropanol is subsequently allowed to cool at a temperature comprised between 0°C and 30°C, which gives rise to VA-2914 isopropanol hemisolvate crystal formation.

Separation of VA-2914 isopropanol hemisolvate crystals is carried out by filtration. Converting VA-2914 isopropanol hemisolvate into VA-2914 can be performed by any conventional method, for example, by recrystallisation in a suitable solvent. In a particular embodiment, VA-2914 is obtained by recrystallising isopropanol hemisolvate in a solvent chosen between ethanol/water and ethyl ether. VA-2914 thus obtained has been identified by its infrared (IR) spectrum, its exotherm by differential scanning calorimetry (DSC) and by its X-ray diffraction (XRD) diffractogram and the results obtained [see Figures 4-7] correspond to those of the VA-2914 crystalline form obtained by recrystallisation in ethyl ether or in ethanol/water, according to the process disclosed in US patent 5,929,262.

### VA-2914 isopropanol hemisolvate

The VA-2914 crystalline form, specifically VA-2914 isopropanol hemisolvate, has been identified and characterised by IR spectroscopy, DSC and XRD.

This VA-2914 crystalline form is an isopropanol hemisolvate, as has been verified by gas chromatography, and has an isopropanol content of about 5.9% by weight [see Example 5].

VA-2914 isopropanol hemisolvate is characterised in that
it shows a potassium bromide pellet IR spectrum substantially similar to that shown in Figure 1, having significant bands at 1684, 1660, 1609, 1595, 1560, 1543, 1513, 1476, 1458, 1438, 1394, 1364, 1353, 1317, 1303, 1260, 1235, 1214, 1201, 1168, 1137, 1089, 1076, 1063, 1042, 1015, 965, 949, 922, 863, 830, 822, 795, 771, 734, 699, 668, 642, 617, 608, 592, 574, 537, 495 and 467 cm⁻¹;
exotherm recording by DSC shows a peak at about 156°C, which corresponds with the endothermic melting phenomenon of said crystalline form at high temperatures (see Figure 2) [DSC recording was performed in a closed vessel, at a temperature comprised between 10°C and 200°C, with a heating rate of 10°C/min, in Mettler Toledo Star System equipment]; and
it shows an X-ray diffraction (XRD) diffractogram (powder) substantially similar to that shown in Figure 3, with characteristic peaks at 8.860, 9.085 and 16.375 degrees 20, using a radiation source with a wavelength α₁ of 1.54060 Å, a wavelength α₂ of 1.54439 Å, a wavelength intensity ratio α₁/α₂ of 0.5, 40 kV voltage and 30 mA current intensity, in Debye-Scherrer INEL CPS-120 equipment; more specifically, XRD analysis of said crystalline form (powder) shows the characteristics listed in Table 1.

**Table 1**

| **VA-2914 Isopropanol Hemisolvate XRD (Powder) Characteristics** | | | | |
|---|---|---|---|---|
| **2θ** | **d α₁ (Å)** | **d α₂ (Å)** | **Peak I. (counts)** | **Rel.I. (%)** |
| 9.085 | 9.7262 | 9.7501 | 10920 | 100.0 |
| 8.860 | 9.9727 | 9.9972 | 6131 | 56.1 |
| 16.375 | 5.4089 | 5.4222 | 5868 | 53.7 |
| 17.750 | 4.9929 | 5.0052 | 5388 | 49.3 |
| 18.720 | 4.7363 | 4.7480 | 4830 | 44.2 |

| | | | | |
|---|---|---|---|---|
| d: distance; Peak I.: Peak Intensity; Rel. I..: Relative Intensity | | | | |

IR, DSC and XRD study and characterisation of VA-2914 isopropanol hemisolvate and its comparison to corresponding analyses by IR, DSC and XRD of VA-2914 compound obtained by recrystallisation in ethyl ether or in ethanol/water [US 5,929,262], has shown that said isopropanol hemisolvate is a VA-2914 crystalline form which is not disclosed in the aforementioned document.

The VA-2914 compound obtained by recrystallisation in ethyl ether or in ethanol/water [US 5,929,262] shows the following characteristics:
it shows a potassium bromide pellet IR spectrum substantially similar to that shown in Figure 4, having significant bands at 1684, 1661, 1611, 1595, 1560, 1542, 1517, 1499, 1458, 1438, 1390, 1364, 1350, 1304, 1253, 1236, 1202, 1167, 1147, 1077, 1064, 1023, 965, 952, 921, 867, 832, 809, 767, 699, 668, 615, 575, 540 and 495 cm⁻¹;
exotherm recording by DSC shows a peak at 189°C, which corresponds with the endothermic melting phenomenon of said crystalline form at high temperatures (see Figure 5) [said DSC recording was performed in a closed vessel, at a temperature comprised between 10°C and 200°C, with a heating rate of 10°C/min, in Mettler Toledo Star System equipment]; and
it shows an X-ray diffraction (XRD) diffractogram substantially similar to that shown in Figure 6, with characteristic peaks at 9.110 and 16.965 degrees 2θ, using a radiation source with a wavelength α₁ of 1.54060 Å, a wavelength α₂ of 1.54439 Å, a wavelength intensity ratio α₁/α₂ of 0.5, 40 kV voltage and 30 mA current intensity, in Debye-Scherrer INEL CPS-120 equipment; more specifically, XRD analysis of said crystalline form shows the characteristics listed in Table 2.

**Table 2**

| **VA-2914 XRD (Powder) Characteristics** | | | | |
|---|---|---|---|---|
| **2θ** | **d α₁ (Å)** | **d α₂ (Å)** | **Peak I. (counts)** | **Rel. I. (%)** |
| 9.110 | 9.6996 | 9.7234 | 21054 | 100.0 |
| 16.965 | 5.2221 | 5.2350 | 13502 | 64.1 |
| 15.130 | 5.8511 | 5.8655 | 8705 | 41.3 |
| 15.010 | 5.8976 | 5.9121 | 8668 | 41.2 |
| 17.165 | 5.1617 | 5.1744 | 8263 | 39.2 |

| | | | | |
|---|---|---|---|---|
| d: distance; Peak I.: Peak Intensity; Rel. I..: Relative Intensity | | | | |

Additionally, an exotherm recording by DSC was simultaneously performed for both crystalline forms [VA-2914 isopropanol hemisolvate and VA-2914 obtained by recrystallisation in ethyl ether or in ethanol/water (US 5,929,262)]. The results obtained are shown in Figure 7, where it can be observed that said DSC recording shows 2 peaks, one at about 156°C, corresponding to the endothermic melting phenomenon of the VA-2914 isopropanol hemisolvate crystalline form at a high temperature, and another one at 189°C, corresponding to the endothermic melting phenomenon of the VA-2914 crystalline form obtained by recrystallisation in ethyl ether or in ethanol/water (US 5, 929, 262), at a high temperature. DSC recording was performed in a closed vessel, at a temperature comprised between 10°C and 200°C, with a heating rate of 10°C/min, in Mettler Toledo Star System equipment.

IR spectroscopy has proven to be a very useful tool for differentiating between both crystalline forms given that very clear and characteristic bands occur for each one of them at wavelengths in which no other band of the other crystalline form occurs. Thus, for example, the band occurring at 809 cm⁻¹ in the IR spectrum of the VA-2914 compound obtained by recrystallisation in ethyl ether or in ethanol/water (US 5,929,262) [Figure 4] does not occur in the IR spectrum of VA-2914 isopropanol hemisolvate [Figure 1].

XRD powder characterisation of both crystalline forms [VA-2914 isopropanol hemisolvate and VA-2914 obtained by recrystallisation in ethyl ether or in ethanol/water (US 5,929,262)] is significantly different, which shows the existence of two different crystalline forms. As is known, X-Ray studies form the best tool for differentiating between two crystalline forms given that each crystalline form has different and characteristic diffractions, and, therefore, each form can be unequivocally identified. The simple comparison of the XRDs for said crystalline forms shows that there are diffractions in one of the crystalline forms which fall in an area where there are no diffractions of the other crystalline form [sees Figures 3 and 6, and Tables 1 and 2]

The previously mentioned data confirms that the VA-2914 isopropanol hemisolvate provided by this invention is different to the VA-2914 crystalline form obtained by recrystallisation in ethyl ether or in an ethanol/water mixture, according to the process described in US patent 5. 929.262.

VA-2914 isopropanol hemisolvate can be obtained by means of a process comprising dissolving VA-2914 in isopropanol under heat, for example, at a temperature comprised between 75°C and the solvent reflux temperature, and allowing the resulting solution to cool down to a temperature comprised between 0°C and 30°C. This VA-2914 crystalline form can be used as an intermediate in obtaining highly pure VA-2914 or in purifying raw VA-2914.

### Carbinol acetate

Compound 3,3-(1,2-ethanedioxy)-5α-hydroxy-11β-(4-N,N-dimethylaminophenyl)-17α-acetoxy,19-norpregna-9-ene-20-one, called carbinol acetate in this description, with formula 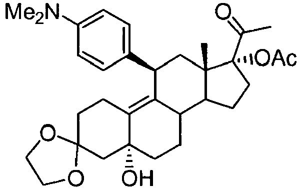 is useful in synthesising VA-2914.

Carbinol acetate can be obtained by means of a process such as that shown in the following Reaction Scheme.

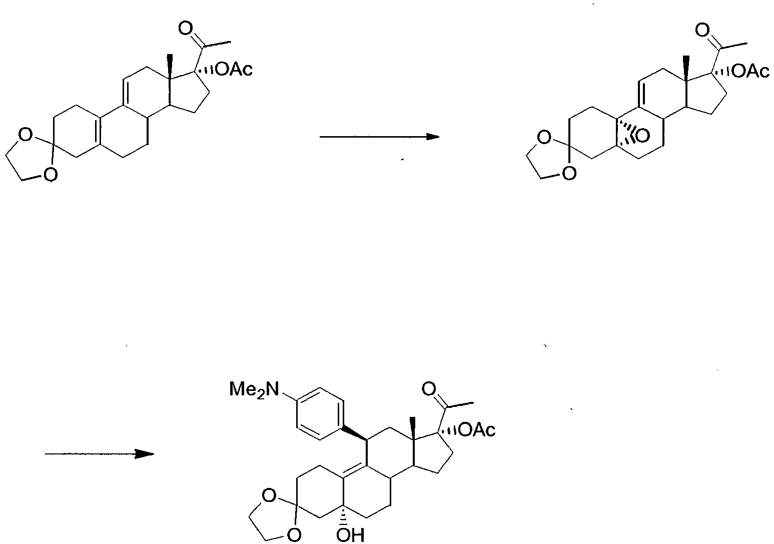

Briefly, obtaining carbinol acetate comprises, in a first step, epoxidising the double bond 5 (10) present in 17α-acetoxy-3,3-(1,2-ethanedioxy)-19-norpregna-5,(10),9(11)-diene-20-one by means of reacting said compound with an adduct formed by reaction of a halogenated ketone and a peroxide, in the presence of a base and a solvent. Said halogenated ketone can be a halogenated acetone, for example, hexafluoroacetone or hexachloroacetone. Any suitable peroxide can be used in this reaction, for example, hydrogen peroxide, an alkaline metal peroxide, a peroxyacid, etc. This epoxidation reaction is carried out in the presence of a base, preferably of an inorganic base, such as, for example, a phosphate, an alkaline metal carbonate or bicarbonate and an organic solvent, preferably a halogenated solvent. In a particular embodiment, the halogenated ketone hexafluoroacetone, the peroxide is hydrogen peroxide, the base is dibasic sodium phosphate and the solvent is dichloromethane.

The epoxide obtained is then reacted in a second step with a Grignard reagent, such as 4-N,N-dimethylaminophenylmagnesium bromide in the presence of a Cu(I) salt in order to obtain carbinol acetate.

The following Examples illustrate the invention and must not be considered to be limiting thereof.

### EXAMPLE 1

### Obtaining raw 17β-acetoxy-11β-(4-N,N-dimethylaminophenyl)-19-norpregna-4,9-diene-3,20-dione [VA-2914]

38.5 g of purified 3,3-(1,2-etthanedioxy)-5α-hydroxy-11β-(4-N,N-dimethylaminophenyl)-17α-acetoxy-19-norpregna-9-ene-20-one [carbinol acetate] were loaded into a flask under nitrogen atmosphere at a temperature comprised between 20°C and 22°C, and 385 ml of deionised water and 17.91 g of HKSO₄ were added. The obtained suspension was stirred until complete dissolution, for about 4 hours. The end of the reaction was determined by means of thin layer chromatography (TLC).

3.85 g of neutral Al₂O₃ were then added, it was stirred for 30 minutes, the suspension was filtered and the insoluble particles were washed with 38.5 ml of deionised water. 325 ml of ethyl acetate were added to the filtrates and the pH was adjusted to a constant value between 7.0 and 7.2 with 7% w/v sodium bicarbonate solution. The phases were allowed to decant for 15 minutes and, after verifying the absence of the final product therein by means of TLC, the phases were separated, discarding the aqueous phase.

192.5 ml of deionised water were added to the resulting organic phase, it was stirred for 10 minutes and the phases were allowed to decant for 15 minutes. After verifying the absence of the final product in the aqueous phase by means of TLC, the phases were separated, discarding the aqueous phase.

The resulting organic phase was vacuum-concentrated until obtaining a residue and about 28 g of raw 17α-actoxy-11β-(4-N,N-dimethylaminophenyl)-19-norpregna-4,9-diene-3,20-dione [VA-2914] were obtained.

### EXAMPLE 2

### Obtaining 17α-acetoxy-11β-(4-N,N-dimethylaminophenyl)-19-norpregna-4,9-diene-3,20-dione isopropanol hemisolvate

2 x 38.5 ml of isopropanol vacuum-concentrated to a residue both times were added to the raw 17α-acetoxy-11β-(4-N,N-dimethylaminophenyl)-19-norpregna-4,9-diene-3,20-dione obtained in Example 1. 77 ml of isopropanol were added to the obtained solid and it was heated until dissolution. It was then allowed to cool to a temperature comprised between 0°C and 5°C, and the temperature was maintained for 1 hour. The obtained suspension was filtered and the cake was washed with cold isopropanol. The yield achieved was 96% molar (5.5% isopropanol content).

The VA-2914 isopropanol hemisolvate obtained was characterised by IR spectroscopy, DSC and XRD, as indicated in the description, and has the characteristics indicated therein and shown in Figures 1-3.

### EXAMPLE 3

### Converting 17α-acetoxy-11β-(4-N,N-dimethylaminophenyl)-19-norpregna-4,9-diene-3,20-dione isopropanol hemisolvate into VA-2914

10 g of VA-2914 isopropanol hemisolvate were suspended under stirring in 100 ml of an ethanol/H₂O (80/20) mixture. The suspension is heated until dissolution and once the product is dissolved said solution is cooled at 15-20°C. The crystals obtained are separated by filtration and the product is dried in a vacuum oven until achieving a constant weight. 7.5 g are obtained of VA-2914 of the desired crystalline form, which has been characterised by IR spectroscopy, DSC and XRD, and shows the characteristics of the VA-2914 compound obtained by recrystallisation in ethyl ether or in ethanol/water (US 5,929,262) and shown in Figures 4-6, are obtained.

### EXAMPLE 4

### Obtaining 3,3-(1,2-ethanedioxy)-11β-(4-N,N-dimethylaminophenyl)-17α-acetoxy-19-norpregna-9-ene-20-one [Carbinol acetate]

### Step 1:

### Synthesising 17α-Acetoxy-3,3-(1,2-ethanedioxy)-5,10α-epoxy-19-norpregna-9(11)-ene-20-one

14.82 g of 17α-acetoxy-3,3-(1,2-ethanedioxy)-19-norpregna-5,(10),9(11)-diene-20-one (37 mmol) are dissolved in 220 ml of Cl₂CH₂ and the resulting solution is cooled at 0°C. 3.15 g of Na₂HPO₄ (22.24 mmol), 3.1 ml of hexafluoroacetone (22.24 mmol) and 5.3 ml of 50 % H₂O₂ (91.9 mmol) are added. The mixture is heated at room temperature and is stirred overnight. The reaction is hydrolysed with a saturated solution of NaHCO₃ and it is extracted 3 times with Cl₂CH₂. The pooled organic phases are dried with Na₂SO₄ and vacuum-concentrated until completely eliminating the solvent. A yellow solid is obtained with a quantitative yield. The raw solid obtained is a 4:1 mixture of the 9(10) α and β epoxy isomers. The crude obtained is used in the following step without purifying.

### Step 2

### Synthesising 3,3-(1,2-Ethanedioxy)-5α-hydroxy-11β-(4-N,N-dimethylaminophenyl)-17α,-acetoxy-19-norpregna-9-ene-20-one

The residue from the previous step is dissolved in 150 ml of dry THF under nitrogen atmosphere and 4.2 g of ClCu are added. The suspension is cooled at 0°C and 92.5 mmol of a solution of 4-N,N-dimethylaminophenylmagnesium bromide in freshly prepared THF are added. After 10 minutes the mixture is hydrolysed with 200 ml of a saturated solution of NH₄Cl, the mixture is stirred for 5 minutes at room temperature and the phases are decanted. The resulting organic phase is vacuum-concentrated until eliminating all the solvents. The obtained residue is purified by column chromatography and 12.5 g (63.0 %) of the title compound are obtained.
¹H NMR (400 MHz, CDCl₃) δ 7.0 (dd, 2, ArH), 6.62 (dd, 2, ArH), 4.41 (s, 1, -OH, C₅) 4.28 (d, 1, CH, C₁₁), 3.9 (m, 4, cetal (CH₂)₂, C₃), 2.85 (s, 6, -N(CH₃)₂), 2.12 (s, 3, CH₃, C₂₀), 2.07 (s, 3, acetate CH₃, C₁₇), 0.25 (s, 3, CH₃, C₁₈).

### EXAMPLE 5

### Determining isopropanol content in 17α-acetoxy-11β-(4-N,N-dimethylaminophenyl)-19-norpregna-4,9-diene-3,20-dione isopropanol hemisolvate

Determining isopropanol content in 17α-acetoxy-11β-(4-N,N-dimethylaminophenyl)-19-norpregna-4,9-diene-3,20-dione (VA-2914) isopropanol hemisolvate was performed by means of gas chromatography in a 5% phenyl methyl silicone column (30 m), using nitrogen as a carrier gas.

Isopropanol detection was carried out by means of flame ionisation; at an isothermal oven temperature of 65°C. Detector temperature was 300°C and injector temperature was 250°C.

Analysis was carried out by means of the internal standard technique. To this end a solution with a concentration of 50 mg of dioxane per ml of dimethylformamide (DMF) ["Internal STD Solution"] was prepared. A solution of 50 mg of isopropanol/ml of DMF was prepared as a "Standard Preparation". 1 ml of each one of the previous solutions ("Internal STD Solution" and "Standard Preparation") were then taken and diluted to a volume of 10 ml with DMF.

To carry out this test, 1 g of VA-2914 isopropanol hemisolvate was weighed, 1 ml of the "Internal STD Solution" was added and it was diluted to a volume of 10 ml with DMF ("Test Preparation").

The "Test Preparation" and the "Standard Preparation" were injected in the gas chromatographer and the isopropanol content in the VA-2914 isopropanol hemisolvate sample was calculated by means of the internal standard technique. The isopropanol content is 5.9%, which corresponds to the theoretical amount for a VA-2914 isopropanol hemisolvate.

## Claims

1. A process for purifying 17α-acetoxy-11β-(4-N,N-dimethylaminophenyl)-19-norpregna-4,9-diene-3,20-dione (VA-2914) comprising:
a) forming VA-2914 isopropanol hemisolvate crystals by means of crystallising VA-2914 in isopropanol;
b) separating the VA-2914 isopropanol hemisolvate crystals by filtration; and
c) converting VA-2914 isopropanol hemisolvate into VA-2914.

2. A process according to claim 1, wherein formation of VA-2914 isopropanol hemisolvate crystals comprises dissolving VA-2914 in isopropanol under heat, and subsequent cooling of the resulting solution, optionally under stirring.

3. A process according to claim 2, wherein the VA-2914 and isopropanol mixture is heated at a temperature comprised between 75°C and the solvent reflux temperature, until complete dissolution of VA-2914, and subsequently, the resulting solution of VA-2914 in isopropanol is allowed to cool at a temperature comprised between 0°C and 30°C.

4. A process according to claim 1, wherein conversion of VA-2914 isopropanol hemisolvate into VA-2914 is carried out by recrystallisation in a solvent.

5. A process according to claim 4, wherein conversion of VA-2914 isopropanol hemisolvate into VA-2914 is carried out by recrystallisation in a solvent chosen between ethanol/water and ethyl ether.

6. A process according to claim 1, wherein said VA-2914 compound is obtained by acid hydrolysis of compound 3,3-(1,2-ethanedioxy)-5α-hydroxy-11β-(4-N,N-dimethylaminophenyl)-17α-acetoxy-19-norpregna-9-ene-20-one [carbinol acetate].

## Patentansprüche

1. Ein Verfahren zur Aufreinigung von 17α-Acetoxy-11β-(4-N,N-dimethylaminophenyl)-19-norpregna-4,9-dien-3,20-dion (VA-2914) enthaltend:
a) Bilden von VA-2914 Isopropanol-Hemisolvat-Kristallen durch Kristallisation von VA-2914 in Isopropanol;
b) Abtrennen der VA-2914 Isopropanol-Hemisolvat-Kristalle durch Filtration; und
c) Umwandeln des VA-2914 Isopropanol-Hemisolvats in VA-2914.

2. Ein Verfahren gemäß Anspruch 1, wobei die Bildung von VA-2914 Isopropanol-Hemisolvat-Kristallen das Lösen von VA-2914 in Isopropanol unter Erwärmung, und anschließendem Abkühlen der erhaltenen Lösung, optional unter Rühren, umfasst.

3. Ein Verfahren gemäß Anspruch 2, wobei die Mischung von VA-2914 und Isopropanol auf eine Temperatur erhitzt wird, die zwischen 75°C und der Refluxtemperatur des Lösungsmittels liegt, bis eine vollständige Lösung von VA-2914 erreicht wird, und anschließend, die erhaltene Lösung von VA-2914 in Isopropanol abgekühlt wird auf eine Temperatur zwischen 0°C und 30°C.

4. Ein Verfahren gemäß Anspruch 1, wobei die Umwandlung des VA-2914 Isopropanol-Hemisolvats in VA-2914 durch Rekristallisation in einem Lösungsmittel durchgeführt wird.

5. Ein Verfahren gemäß Anspruch 4, wobei die Umwandlung des VA-2914 Isopropanol-Hemisolvats in VA-2914 durch Rekristallisation in einem Lösungsmittel ausgewählt aus Ethanol/Wasser und Ethylether durchgeführt wird.

6. Ein Verfahren gemäß Anspruch 1, wobei die Verbindung VA-2914 durch saure Hydrolyse der Verbindung 3,3-(1,2-Ethandioxy)-5α-hydroxy-11β-(4-N,N-dimethylaminophenyl)-17α-acetoxy-19-norpregna-9-en-20-on [Carbinolacetat] erhalten wird.

## Revendications

1. Procédé de purification de 17α-acétoxy-11β-(4-N,N-diméthylaminophényl)-19-norprégna-4,9-diène-3,20-dione (VA-2914)comprenant :
a) la formation de cristaux d'hémisolvate d'isopropanol de VA-2914 en cristallisant le VA-2914 dans de l'isopropanol ;
b) la séparation des cristaux d'hémisolvate d'isopropanol de VA-2914 par filtration ; et
c) la conversion de l'hémisolvate d'isopropanol de VA-2914 en VA-2914.

2. Procédé selon la revendication 1, dans lequel la formation de cristaux d'hémisolvate d'isopropanol de VA-2914 comprend la dissolution du VA-2914 dans de l'isopropanol sous l'effet de la chaleur, et un refroidissement ultérieur de la solution résultante, facultativement sous agitation.

3. Procédé selon la revendication 2, dans lequel le mélange de VA-2914 et d'isopropanol est chauffé à une température comprise entre 75°C et la température de reflux du solvant, jusqu'à dissolution totale du VA-2914, et ensuite, la solution résultante de VA-2914 dans l'isopropanol est mise à refroidir à une température comprise entre 0°C et 30°C.

4. Procédé selon la revendication 1, dans lequel la conversion de l'hémisolvate d'isopropanol de VA-2914 en VA-2914 est réalisée par recristallisation dans un solvant.

5. Procédé selon la revendication 4, dans lequel la conversion de l'hémisolvate d'isopropanol de VA-2914 en VA-2914 est réalisée par recristallisation dans un solvant choisi entre l'éthanol/eau et l'éther éthylique.

6. Procédé selon la revendication 1, dans lequel ledit composé de VA-2914 est obtenu par l'hydrolyse acide du composé 3,3-(1,2-éthanedioxy)-5α-hydroxy-11β-(4-N,N-diméthylaminophényl)-17α-acétoxy-19-norprégna-9-ène-20-one [acétate de carbinol].
